# EUROPEAN PATENT APPLICATION

(11) **EP 4 549 561 A1**
(43) Date of publication of application: **07.05.2025**
(21) Application number: 23207190.2
(22) Date of filing: 31.10.2023
(51) Int. Cl.: C12N 9/20, C12N 11/08, C12N 11/091, C12P 7/6458

(54) **PROCESS FOR IMMOBILIZATION OF LIPASES**

(71) Applicant: Frutarom Ltd., 2310001 Migdal Haemek (IL)
(72) Inventor: LAOZ, Hala, 15235 Kfar Kama (IL); BARENBOIM, Hen, 18043 Afula (IL)
(74) Representative: J A Kemp LLP

(57) **Abstract**

The invention provides a process for immobilizing a lipase on a solid support, the support containing functional amine group/s, the process comprising a step of contacting the lipase with said support in an aqueous reaction medium in the presence of an ionic surfactant agent.

The invention also provides an immobilized lipase prepared by the process, the use of an ionic surfactant in a process for immobilizing a lipase on a solid support, and a method for producing a structured triglycerides product by enzymatic transesterification of an oil source.

## Description

### TECHNICAL FIELD

Disclosed herein is a process of immobilizing a lipase on a support having a functional amine group in the presence of a surface-active material which is an ionic surfactant.

### BACKGROUND

Lipases (E.C. 3.1.1.3) are enzymes that break down triglycerides into partial glycerides, mono- and di-glycerides, free fatty acids and glycerol by the hydrolysis of the ester bonds in triglycerides. Different lipases can exhibit different regio-specificity with respect to the glycerol backbone.

Immobilized enzymes are enzymes physically or chemically attached to an inert solid support, while retaining their catalytic activity. Unlike free enzymes, immobilized enzymes can be separated and used repeatedly and are therefore able to catalyze reactions continuously. In addition, immobilization is an efficient way to improve the enzymatic features, including activity, selectivity, and stability, for example by facilitating accessibility of the enzyme active site.

EP3146044 describes a process for immobilizing a lipase on a support containing a functional amino group in the presence of a surface-active material in an aqueous solution, wherein the surfactant is a non-ionic surfactant and functional amine group refers to an amine group which is engaged in interacting with or binding to the lipase and optionally the support.

### SUMMARY OF INVENTION

Disclosed herein is a process for immobilizing a lipase on a solid support, the support containing functional amine group/s, the process comprising a step of contacting the lipase with said support in an aqueous reaction medium in the presence of an ionic surfactant agent.

In some or all embodiments of the present disclosure, the said surfactant agent is a fatty acid, specifically a fatty selected from C6-C22 fatty acids, C8-C20 fatty acids, C12-C18 fatty acids, and C16-C18 fatty acids, more specifically an unsaturated or monosaturated C16-C18 fatty acid, suitably C18 such as stearic acid or oleic acid, preferably oleic acid.

In some or all embodiments of the present disclosure, the said support is an ion exchange support.

In some or all embodiments of the present disclosure, the said support is a crosslinked phenol-formaldehyde polycondensate, preferably crosslinked phenol-formaldehyde polycondensate containing a tertiary amine group.

In some or all embodiments of the present disclosure, said surfactant agent is contained in said reaction medium at a concentration of at least about 0.01wt%, at least about 0.1wt%, at least about 1wt%, and up to about 15wt%.

In some or all embodiments of the present disclosure, the weight ratio between said surfactant agent and said solid support is about 0.1-0.5, about 0.15-0.375, about 0.15 or about 0.375.

In some or all embodiments of the present disclosure, the lipase to support weight ratio in the immobilization reaction mixture is about 0.3-2, about 0.5-1.5, about 0.7-1.2, about 0.7, about 1, or about 1.2.

In some or all embodiments of the present disclosure, the said lipase is derived from *Aspergillus oryzae* (expressing the lipase gene from *Thermomyces lanuginosus*) or is derived from *Rhizopus oryzae.*

In some or all embodiments of the present disclosure, the reaction mixture comprising said reaction medium, said support, said surfactant agent and said lipase, is maintained at room temperature for a suitable period of time, optionally whilst mixing.

In some or all embodiments of the present disclosure, the resulting immobilized lipase is isolated from the reaction medium and is dried under reduced pressure at 35-50 ⁰C, preferably to a water content of up to about 6-8wt.%.

Further disclosed herein is an immobilized lipase prepared by the said process of immobilization.

In a further embodiment disclosed herein is use of an ionic surfactant in a process for immobilizing a lipase on a solid support in an aqueous reaction medium in the presence of the ionic surfactant, the process comprising the step of drying the immobilized lipase, wherein the use of the ionic surfactant provides a reduction in the drying time of the immobilized lipase and/or promotes drying of the immobilized lipase.

The said ionic surfactant used is a fatty acid, specifically a fatty selected from C6-C22 fatty acids, C8-C20 fatty acids, C12-C18 fatty acids, and C16-C18 fatty acids, more specifically an unsaturated or monosaturated C16-C18 fatty acid, suitably C18 such as stearic acid or oleic acid, preferably oleic acid.

In the process of said use, said support is an ion exchange support, preferably a crosslinked phenol-formaldehyde polycondensate, wherein said crosslinked phenol-formaldehyde polycondensate optionally contains a tertiary amine group.

Further, in the process of said use, said lipase is a lipase derived from *Aspergillus oryzae* (expressing the lipase gene from *Thermomyces lanuginosus*) or derived from *Rhizopus oryzae.*

Further disclosed herein is an immobilized lipase prepared by the process of said use of an ionic surfactant.

In another aspect, disclosed herein is a method for producing a structured triglycerides product by enzymatic transesterification of an oil source, using at least one lipase immobilized on a solid support containing functional amine groups prepared by the said process of immobilization, or by the process of said use of an ionic surfactant.

In said method the said structured triglycerides product can be a beta-palmitate triglycerides product, but is not limited thereto.

The said beta-palmitate triglycerides product can be an oleic-palmitic-oleic (OPO) triglycerides product.

The said structured beta-palmitate triglycerides product can contain, for example, from about 15% to about 55% of palmitic acid moieties out of the total fatty acid moieties of the beta-palmitate triglycerides.

In some embodiments, the content of the palmitic acid moieties at the sn-2 position of said beta-palmitate triglycerides product is at least 30%, at least 33%, at least 38%, at least 40%, at least 45%, at least 50%, at least 52% or at least 60% out of the total palmitic acid moieties in the beta-palmitate triglycerides product.

In some embodiments of said method of preparing structured triglycerides, the content of triglycerides that contain a total of 52 carbon atoms (C52 triglycerides) in the structured beta-palmitate triglycerides product is at least 13%, at least 15%, at least 20%, at least 25%, at least 30%, at least 35%, at least 40% or at least 50%.

### BRIEF DESCRIPTION OF THE FIGURES

In order to better understand the subject matter disclosed herein and to exemplify how it can be carried out in practice, embodiments will be described, by way of non-limiting example only, with reference to the accompanying drawings, in which:
**Figure 1** illustrates a comprison between drying rates of immobilized lipases produced using non-ionic vs. ionic surfactants at surfactant: resin ratio of 0.15.
**Figure 2** illustrates a comparison between the enzymatic activity of immobilized lipases produced using non-ionic vs ionic surfactants at surfactant: resin ratio of 0.15.
**Figure 3** illustrates a comparison between drying rates of immobilized lipases produced using non-ionic vs ionic surfactant at surfactant: resin ratio of 0.375.
**Figure 4** illustrates a comparison between the enzymatic activity of immobilized lipases produced using non-ionic vs ionic surfactants at surfactant: resin ratio of 0.375.

### DESCRIPTION OF EMBODIMENTS

Disclosed herein is a process for immobilizing a lipase on a solid support, which enables shorter drying time of the immobilized lipase preparation following the immobilization reaction.

The drying process is carried out at high temperatures for a substantial time duration and during this time, the lipase is exposed to high temperature and to moisture that may adversely affect its activity. Therefore, reducing drying time may contribute to the properties of the enzyme, such as stability, productivity and activity.

Additionally, reducing the duration of drying stage of the production process has environmental and commercial benefits since it results in energy saving and shorter production time.

In a first aspect the present disclosure relates to a process of immobilizing a lipase on a solid support, the support containing a functional amine group, wherein the lipase is brought into contact with the solid support in an aqueous reaction mixture comprising said lipase and said solid support, and an ionic surfactant. The reaction mixture is maintained whilst stirring at room temperature for a suitable period of time, and then the immobilized lipase preparation is separated off, for example by centrifuging the reaction mixture, and dried.

The present inventors found that immobilization of a lipase on a support containing a functional amine group in the presence of an ionic surfactant has benefits over immobilization of a lipase using an alternative surfactant. The advantages of this finding are elaborated on and exemplified below.

According to the present disclosure, the immobilized lipase is any lipase capable of catalyzing hydrolysis/esterification of triglycerides and/or partial glycerides such as di- and monoglycerides, or esterification of glycerol with fatty acid moieties. Interchanging ester groups between oil or fat triglyceride molecules enables obtaining oils/fats with desired composition and properties.

A lipase as referred to herein may be regio-specific, with position specificity to the sn-1,3 positions of the glycerol backbone. Further, a lipase as referred to herein may have specificity toward certain type of fatty acids, such as short-, medium or long-chain fatty acids, and/or saturated and unsaturated fatty acids. Lipases according to the present disclosure and their immobilizing supports meet food grade requirements.

Specific lipases immobilized according to the present disclosure may be, but are not limited to, lipase derived from Aspergillus Niger, lipase from a genetically modified strain of *Penicillium Camemberti,* lipase derived from *Aspergillus Oryzae,* expressing the lipase gene from *Thermomyces lanuginosus,* lipase derived from *Rhizopus oryzae,* lipase derived from *Candida Rugosa,* lipase derived from a modified strain of *S. Cerevisiae,* lipase derived from *Penicillium Roqueforti,* lipase derived from *Candida Cylindracea,* lipase derived from *Burkholderia Ubonensis,* lipase derived from a genetically modified strain of *Aspergillus Niger* aggregate, lipase derived from *Mucor Javanicus,* or lipase derived from *Rubus Niveus.*

The support on which the lipase is immobilized can be any suitable solid support, such as polymeric support, for example, an ion exchange resin such as a weak, medium or strong anion exchange resin or a weak, medium or strong cation exchange resin in the form of, for example, granulated powder or solid beads.

In specific embodiments, the support on which the lipase is immobilized may be an anion exchange resin, which contains a functional amine group. Specific exemplary supports may be, but are not limited to, supports comprising methyl methacrylate units (methacrylic polymer) or crosslinked phenol-formaldehyde polycondensate, highly cross-linked long chain alkyl acrylate polymer, divinylbenzene copolymer or silicon dioxide. An example of a long chain alkyl acrylate is C₁₈-alkyl acrylate.

The functional amine groups comprised in the support may be primary amine, secondary amine, tertiary amine or quaternary amine groups. The functional amine groups may optionally be selected from amine epoxy, alkyl amine, such as short-chain, e.g. ethyl amine, medium-chain, e.g. C₆-C₁₀ alkyl amine, or long-chain alkyl amine. In some embodiments, the functional amine groups are tertiary amines.

The ionic surfactant may be any suitable ionic surfactant, such as, but not limited to a fatty acid. The fatty acid may be saturated, monounsaturated, or polyunsaturated fatty acid. The fatty acid may be selected from C6-C22 fatty acids, C8-C20 fatty acids, C12-C18 fatty acids, and C16-C18 fatty acids. In some embodiments the ionic surfactant is an unsaturated or monosaturated C16-C18 fatty acid, suitably C18. In some embodiments the ionic surfactant is oleic acid or stearic acid.

In the presently disclosed lipase immobilization process, the surfactant concentration in the immobilization reaction mixture may be at least about 0.01wt%, at least about 0. 1wt%, at least about 1wt%, and up to about 2, 3, 4, 5, 6, 7, 8, 9, 10, 11, 12, 13, 14 or 15wt%, and any intermediate range therebetween, for example, about 0.01-1, about 0.1-1, about 0.01-2, about 0.1-2, about 0.01-10, about 0.1-10 or about 0.01-15, about 0.1-15, 1-15 etc.

The surfactant to support weight ratio in the immobilization reaction mixture may be, for example, about 0.1-0.5, about 0.15-0.375, about 0.15 or about 0.375. Other lower or higher ratios are contemplated.

The lipase to support weight ratio in the immobilization reaction mixture is generally about 0.3-3, about 0.3-2, about 0.5- 1.5, about 0.7-1.2, about 0.7, about 1 or about 1.2, and any ratio or ratio range therebetween.

In commercially available enzyme immobilization processes, the immobilized enzyme is dried at a temperature of between about 35-50 ⁰C for 72-90 hours. Throughout this period of time, the lipase is exposed to temperature and moisture conditions that may adversely affect its activity. Therefore, reducing the drying time of the immobilized enzyme may improve the enzyme's stability, productivity, and activity. Additionally, decreasing drying time of the immobilized enzyme product has environmental and commercial benefits since it results in energy saving and shorter production time.

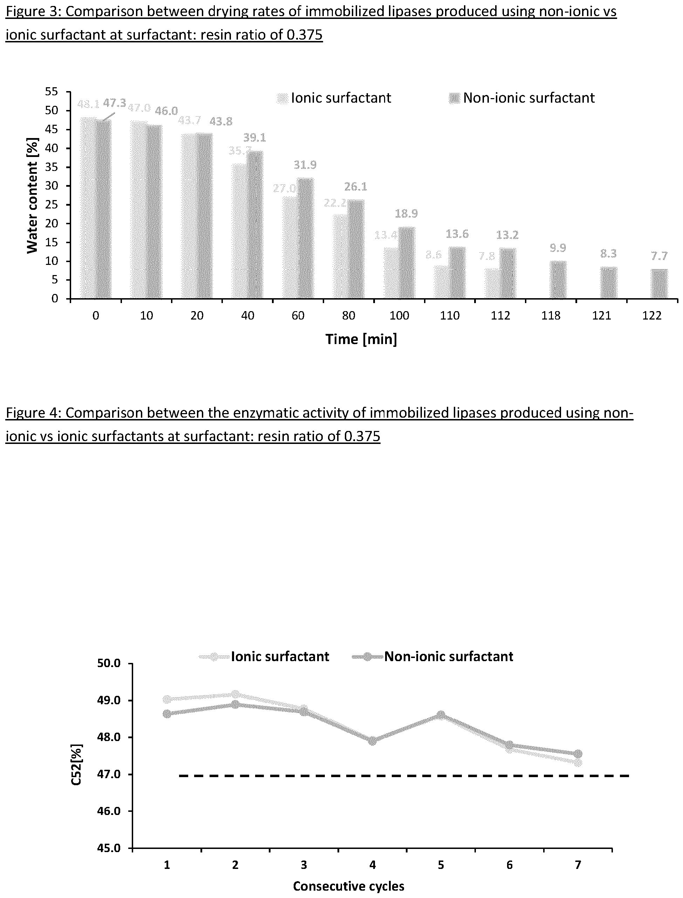

As shown in Fig. 3, using an ionic surfactant during the immobilization reaction enabled reaching the desired water content of the immobilized lipase faster (within about 112 minutes), in comparison to using a non-ionic surfactant (around 122 min). This represents a reduction of about 8% in drying time which is significant in saving production costs and time and in reducing damage to the immobilized enzyme preparation such as reduced enzymatic activity due to prolonged exposure to heat and moisture.

In a further aspect, the present disclosure relates to a use of an ionic surfactant in a process for immobilizing a lipase on a solid support in an aqueous reaction medium in the presence of the ionic surfactant, the process comprising the step of drying the immobilized lipase, wherein use of the ionic surfactant provides a reduction in the drying time of the immobilized lipase and/or promotes drying of the immobilized lipase. The lipase, support and the ionic surfactant are as suitably as disclosed herein. The process is suitably as disclosed herein.

In yet another aspect, the present disclosure relates to a method of enzymatically producing structured triglycerides by enzymatic transesterification of an oil source, using a lipase immobilized on a solid support comprising functional amine groups, wherein said lipase is immobilized on said support in the presence of an ionic surfactant.

The method according to this aspect of the present disclosure comprises subjecting a reaction mixture of an oil source and/or fat source and a fatty acid donor, for example free fatty acids and short-chain alkyl fatty acid esters such as methyl or ethyl fatty acid esters, to enzymatic transesterification in the presence of an immobilized lipase at a suitable temperature, for example, but not limited to about 30 °C to 70 °C, whilst stirring, for a suitable period of time, stopping the reaction by removing the immobilized enzyme and collecting the structured triglycerides produced. The immobilized lipase used in the method is a lipase immobilized according to the present disclosure, namely a lipase immobilized on a solid support comprising functional amine groups, wherein said lipase is immobilized on said support in the presence of an ionic surfactant.

Also in this aspect of the present disclosure the lipases may be, but is not limited to, lipase derived from *Aspergillus Niger,* lipase from a genetically modified strain of *Penicillium Camemberti,* lipase derived from *Aspergillus Oryzae,* expressing the lipase gene from *Thermomyces lanuginosus,* lipase derived from *Rhizopus oryzae,* lipase derived from *Candida Rugosa,* lipase derived from a modified strain of *S. Cerevisiae,* lipase derived from *Penicillium Roqueforti,* lipase derived from Candida *Cylindracea,* lipase derived from *Burkholderia Ubonensis,* lipase derived from a genetically modified strain of *Aspergillus Niger* aggregate, lipase derived from *Mucor Javanicus,* or lipase derived from *Rubus Niveus.*

The oil/fat source used in the method of this aspect of the disclosure is generally, but not limited to, a plant-derived oil or fat, such as, palm oil, palm stearin and/or tripalmitin oil. The fatty acid used in the transesterification reaction is derived from any one of palm kernel oil, sunflower oil and/or high-oleic sunflower oil.

In particular, the disclosed method of preparing structured triglyceride is a method for producing structured beta-palmitate triglycerides, such as OPO (oleic-palmitic-oleic) triglycerides. In a particular embodiment, the oil source is palm stearin, palm oil and/or tripalmitin and the free fatty acid is oleic acid and/or linoleic acid.

The structured beta-palmitate triglycerides product produced by the presently disclosed method, generally contains about 15% to about 55% of palmitic acid moieties out of the total fatty acid moieties of the beta-palmitate triglycerides product.

The content of the palmitic acid moieties at the sn-2 position out of the total palmitic acid moieties in the produced structured beta-palmitate triglycerides product is at least 30%, at least 33%, at least 38%, at least 40%, at least 45%, at least 50%, at least 52% or at least 60%.

The content of triglycerides that contain a total of 52 carbons (C52 triglycerides) in the produced structured beta-palmitate triglycerides product is at least 13%, at least 15%, at least 20%, at least 25%, at least 30%, at least 35%, at least 40% or at least 50%.

"About" as used herein generally refers to approximate values. When referred to a dose of cannabinoids in milligrams, "about" should be understood as including the range of a value ± 15 %. When referred to other values, the term should be understood as including the range of a value ± 15%, for example ± 15%, ± 12%, ± 10%, ± 8%, ± 5%, ± 2% or ± 1%.

As used herein, the term *"**vegetable oil**"* refers to an oil from vegetable sources. The vegetable oil may be a natural vegetable oil or a processed oil from vegetable source. The term *"**vegetable fat**"* refers to a fat from vegetable sources. The vegetable fat may be a natural vegetable fat or a processed fat from vegetable source.

The term "lipase" as used herein also encompasses "phospholipase". Generally, lipase as used herein refers to a naturally occurring lipase enzyme obtained from a natural source by an industrial fermentation process. The terms "obtained from" and "derived from" a natural source may be used herein interchangingly.

As used herein, the term *"and*/*or"* includes any combinations of one or more of the associated listed items. Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

As used in the specification and claims, the forms "a", "an" and "the" include singular as well as plural references unless the context clearly dictates.

Throughout out this disclosure, the word "comprise", and variations such as "comprises" and "comprising", will be understood to imply the inclusion of a stated integer or step or group of integers or steps but not the exclusion of any other integer or step or group of integers or steps.

Disclosed and described, it is to be understood that this invention is not limited to the particular examples, methods steps, and compositions disclosed herein as such methods steps and compositions may vary somewhat. It is also to be understood that the terminology used herein is used for the purpose of describing particular embodiments only and not intended to be limiting since the scope of the present invention will be limited only by the appended claims and equivalents thereof.

Where subject matter is described in conjunction with specific embodiments, it is evident that alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent and patent application was specifically and individually indicated to be incorporated herein by reference. In addition, citation or identification of any reference in this application shall not be construed as an admission that such reference is available as relevant prior art to the presently disclosed subject matter.

### EXAMPLES

### Example 1: exploring the effect of ionic vs. non-ionic surfactants on the drying process and activity of lipase derived from Thermomyces lanuginosus immobilized onto a resin.

### Immobilization procedure:

36g lipase originated from Thermomyces lanuginosus (Novozymes) were dissolved in 180ml potassium phosphate buffer and mixed with 30g water-washed ion exchange resin based on crosslinked phenol-formaldehyde polycondensate containing tertiary amine group and 4.5g or 11.25g surfactant which correspond to surfactant: resin weight ratio of 0.15 and 0.375, respectively. The reaction mixture - comprising the lipase, the support and the ionic surfactant - was mixed at room temperature. After 14-18 hours, the immobilized enzyme was washed with water and filtered. The surfactant was either sorbitan monooleate (non-ionic surfactant) or oleic acid (ionic surfactant).

### Drying process:

60g of each immobilized enzyme (wet basis) were each dried using reduced pressure at 35-50 ⁰C and sampled during the drying process. Drying of each of the tested immobilized preparations started at the same initial conditions of enzyme quantity and water content and went through the same drying conditions to enable head-to- head comparison.

### Enzymatic activity assay:

This assay was used to test the enzymatic interesterification using 2-hours consecutive cycles. 0.8g of immobilized enzyme were weighed and transferred to 20ml glass vial. 6g of melted feed (1:2 palm stearin : free oleic acid (FFA), w/w) were then added and the enzymatic interesterification reaction started at 55°C while shaking at 250 rpm. 20 µl samples were collected every 2hr of the reaction. Carbon number was determined by GC using a method based on AOCS Ce 5.86.

### Results

### a. Comparison between immobilized lipases produced using non-ionic vs. ionic surfactant at surfactant: resin ratio of 0.15

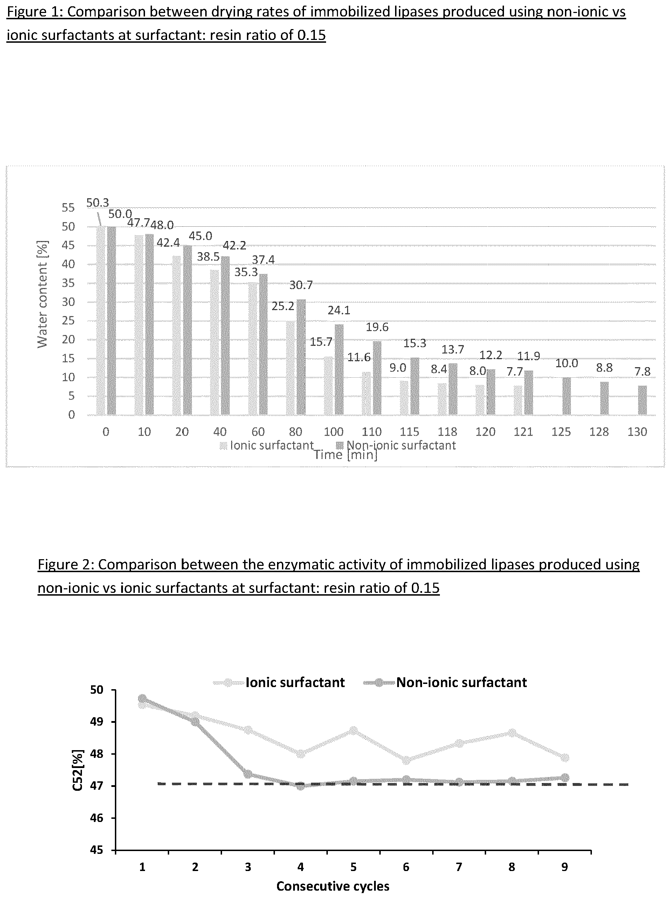

As demonstrated in Figure 1, using an ionic surfactant during the immobilization reaction enables reaching a desired water content (about 7.5-8% as measured by a loss on drying device) faster (within about 120 minutes) in comparison with using a non-ionic surfactant (around 130 min). This represents a reduction of about 8% in drying time which is significant in saving production costs and time and in reducing the chances of harming the enzyme activity due to prolonged exposure to heat and moisture.

As demonstrated in Figure 2, enzymatic activity tests performed following each 2-hour reaction cycle, showed that the use of an ionic surfactant (oleic acid) increased levels of C52 triglycerides (TAGs) over 9 consecutive reaction cycles, exhibiting an advantage over the levels of same TAG using a non-ionic surfactant (sorbitan monooleate).

Thus, by using an ionic surfactant, an improved lipase performance was achieved while using a reduced drying time.

### b. Comparison between immobilized lipases produced using non-ionic vs ionic surfactant at surfactant: resin ratio of 0.375

As shown in Figure 3, also at the exemplified surfactant: resin ratio of 0.375, same as when using a ratio of 0.15 ((a) above), the use of an ionic surfactant during the immobilization reaction enabled reaching the desired water content faster (within about 112 minutes) in comparison with using a non-ionic surfactant (around 122 min), representing a similar reduction of about 8% in drying time.

As demonstrated in Figure 4, the results of the enzymatic activity tests performed following each 2-hour cycle, were similar in the immobilized enzyme produced using non-ionic surfactant and that produced using an ionic surfactant. This is evident by the comparable C52 levels in the different cycles.

Thus, by using an ionic surfactant, an at least comparable lipase performance was achieved while using reduced drying time.

## Claims

1. A process for immobilizing a lipase on a solid support, the support containing functional amine group/s, the process comprising a step of contacting the lipase with said support in an aqueous reaction medium in the presence of an ionic surfactant agent.

2. The process of claim 1, wherein said surfactant agent is a fatty acid, specifically a fatty selected from C6-C22 fatty acids, C8-C20 fatty acids, C12-C18 fatty acids, and C16-C18 fatty acids, more specifically an unsaturated or monosaturated C16-C18 fatty acid, suitably C18 such as stearic acid or oleic acid.

3. The process of claim 1, wherein said fatty acid is oleic acid.

4. The process of claim 1 wherein said support is an ion exchange support.

5. The process of any one of claims 1-4, wherein said support is a crosslinked phenol-formaldehyde polycondensate, which crosslinked phenol-formaldehyde polycondensate optionally contains a tertiary amine group.

6. The process of any one of claims 1-5, wherein:
a) said surfactant agent is contained in said reaction medium at a concentration of at least about 0.01wt%, at least about 0.1wt%, at least about 1wt%, and up to about 15wt%; and/or
b) the weight ratio between said surfactant agent and said solid support is about 0.1-0.5, about 0.15-0.375, about 0.15 or about 0.375; and/or
c) the lipase to support weight ratio in the immobilization reaction mixture is about 0.3- 2, about 0.5- 1.5, about 0.7- 1.2, about 0.7, about 1 or about 1.2; and/or
d) said lipase is derived from *Aspergillus oryzae* (expressing the lipase gene from *Thermomyces lanuginosus*) or is derived from *Rhizopus oryzae.*

7. The process of any one of claims 1-6, wherein the reaction mixture comprising said reaction medium, said support, said surfactant agent and said lipase, is maintained at room temperature for a suitable period of time, optionally whilst mixing; and optionally
wherein the resulting immobilized lipase is isolated from the reaction medium and is dried under reduced pressure at 35-50 °C, preferably to a water content of up to about 6-8wt.%.

8. An immobilized lipase prepared by the process of any one of claims 1-7.

9. Use of an ionic surfactant in a process for immobilizing a lipase on a solid support in an aqueous reaction medium in the presence of the ionic surfactant, the process comprising the step of drying the immobilized lipase, wherein the use of the ionic surfactant provides a reduction in the drying time of the immobilized lipase and/or promotes drying of the immobilized lipase.

10. The use of claim 9, wherein:
a) said ionic surfactant is a fatty acid, specifically a fatty selected from C6-C22 fatty acids, C8-C20 fatty acids, C12-C18 fatty acids, and C16-C18 fatty acids, more specifically an unsaturated or monosaturated C16-C18 fatty acid, suitably C18 such as stearic acid or oleic acid, preferably oleic acid; and/or
b) said support is an ion exchange support, preferably a crosslinked phenol-formaldehyde polycondensate, wherein said crosslinked phenol-formaldehyde polycondensate optionally contains a tertiary amine group; and/or
c) said lipase is derived from *Aspergillus oryzae* (expressing the lipase gene from *Thermomyces lanuginosus*) or is derived from *Rhizopus oryzae.*

11. An immobilized lipase prepared by the process defined in claim 9 or claim 10.

12. A method for producing a structured triglycerides product by enzymatic transesterification of an oil source, using at least one lipase immobilized on a solid support containing functional amine groups prepared by the process of any one of claims 1-7 or by the process defined in claim 9 or claim 10, or a lipase as defined in claim 8 or claim 11.

13. The method of claim 12, wherein said structured triglycerides product is a beta-palmitate triglycerides product; optionally
wherein said beta-palmitate triglycerides product is an oleic-palmitic-oleic (OPO) triglycerides product.

14. The method of claim 12 or claim 13, wherein said structured beta-palmitate triglycerides product contains from about 15% to about 55% of palmitic acid moieties out of the total fatty acid moieties of the beta-palmitate triglycerides; optionally
wherein the content of the palmitic acid moieties at the sn-2 position is at least 30%, at least 33%, at least 38%, at least 40%, at least 45%, at least 50%, at least 52% or at least 60% out of the total palmitic acid moieties in the beta-palmitate triglycerides product.

15. The method of any one of claims 12-14, wherein the content of triglycerides that contain a total of 52 carbon atoms (C52 triglycerides) in the structured beta-palmitate triglycerides product is at least 13%, at least 15%, at least 20%, at least 25%, at least 30%, at least 35%, at least 40% or at least 50%.
